# EUROPEAN PATENT SPECIFICATION

(11) **EP 0 609 084 B1**
(45) Date of publication and mention of the grant of the patent: **24.09.1997**
(21) Application number: 94300614.8
(22) Date of filing: 27.01.1994
(51) Int. Cl.: A61B 17/32

(54) **Powered rotatable curved instrument**
Schwenkbares gekrümmtes Instrument, motorisch betrieben
Instrument courbé orientable actionné par moteur

(30) Priority: 29.01.1993 US 11364
(43) Date of publication of application: 03.08.1994
(73) Proprietor: SMITH & NEPHEW, INC., Memphis, Tennessee 38116 (US)
(72) Inventor: Smith, Graham, Plaistow, New Hampshire 03865 (US)
(74) Representative: Koepsell, Helmut, Dipl.-Ing.

(56) References cited:
- US-A- 4 646 738
- US-A- 5 152 744

## Description

This invention relates to surgical instruments, and in particular to powered arthroscopic surgical instruments.

Powered arthroscopic surgical instruments typically include a rigid, stationary outer tube within which a a rigid inner tube is rotated by a motor. A cutting implement, such as a blade or abrading burr, is disposed on the distal end of the inner tube. Tissue or bone is exposed to the cutting implement through an opening in the distal end of the outer tube, and tissue or bone fragments cut by the rotating blade or burr are drawn through the interior of the inner tube along with irrigating fluid by the use of suction applied at the proximal end of the instrument. Examples of such surgical instruments are described in US Patent Nos. 4203444, 4274414, 4834729 and 4842578, all of which are assigned to the present applicant.

US-A-4 646 738 discloses all the features of the preamble of claim 1.

Some arthroscopic surgical instruments are linear, that is, straight between their proximal and distal ends. Others are curved to facilitate positioning the cutting implement against tissue to be cut without requiring that the instrument be removed from the body and reinserted through an additional puncture. In a curved instrument, a region of the inner tube is flexible to enable the inner tube to accept the curvature imposed by the outer tube while transmitting the torsion applied by the motor to the blade. In both cases, the user changes the orientation of the cutting implement by rotating the instrument.

A general aspect of the invention as defined in claim 1 is a surgical instrument that includes a first member that has an opening in its distal region for admitting tissue and that is rotatable with respect to a base from which the first member extends to allow the rotational orientation of the opening to be selectively changed with respect to the axis of the instrument; a second member is disposed within the first member to transmit force to move a cutting implement disposed at its distal end and cause it to cut tissue that is exposed to the implement through the opening.

Thus according to the invention we provide a surgical instrument disposed generally along an axis, said surgical instrument comprising;
a first member that extends distally from a base and has an opening in a distal region thereof for admitting tissue;
a second member disposed within said first member comprising a distal region that rotates in response to force applied to a proximal region of said second member
   characterised in that said instrument is provided with actuating means disposed at a proximal region of said first member to selectively change a rotational orientation of said first member with respect to said base.

Among other advantages, the invention allows the user to change the angle of attack of the cutting implement (ie. rotational orientation at which the cutting implement is exposed to tissue) by rotating the first member only, without turning the entire instrument. As a result, the user can maintain the instrument in an essentially fixed position, while rotationally varying the locations at which cutting performed. This minimizes the manipulation require of the entire instrument, thereby facilitating the surgical procedure and reducing patient discomfort and the risk of surgical side effects.

Preferred embodiments include the following features.

In a particular useful embodiment, the first member is provided with a bend region that angularly offsets the distal region (and hence the opening) from the axis of the instrument in a selected direction. In other words, the instrument is curved. The curved nature of the instrument allows the user (eg. a surgeon) to position the cutting implement adjacent to tissue and other body material that is relatively difficult to reach with a straight instrument without having to remove and re-introduce the instrument through additional incisions in the body. Because the first member (rather than the entire instrument) is rotated to vary the angle of cutting attack, the cutting implement is maintained in close contact with the tissue being cut at all times.

In the curved embodiment, the first member is relatively flexible, at least in the bend region, to allow the rotational orientation of the opening to be changed without changing the direction of the offset, and at least a portion of the second member that is disposed in the bend region is also relatively flexible to transmit the applied force through the bend region to the cutting implement.

Thus, only the outer member, and not the bend region itself or the remainder of the instrument, is rotated to change the orientation of the opening. Eliminating the need to rotate the entire instrument is particularly useful with a curved instrument, because the distal region of the instrument is on an axis different from that of the remainder of the instrument. As a result, with a curved instrument in which the outer tube is nonrotatable with respect to the remainder of the instrument, the entire instrument must be pivoted or swung about the axis of the distal region of the instrument to rotate the cutting implement opening. By contrast, the present invention allows the instrument to remain in fixed position while the opening for the cutting implement is rotated. This simplifies operation and reduces the trauma to the body.

The first member is relieved in the bend region to provide the relative flexibility. Preferably, the first member is a tube having rigid proximal and distal regions that are connected by the relieved portion. The first member is relieved with a plurality of discrete openings disposed in its walls. The openings are a series of axially spaced, circumferentially extending slots that extend radially into the first member. Adjacent slots extend into the first member from opposite directions. The configuration and orientation of the slots help ensure uniform flexibility while providing the flexible region of the first member with sufficing torsional stiffness to transmit rotation applied by the user at, eg. the base through the bend region to rotate the opening. A pliable sheath (such as a shrink-wrap tube) may be disposed over the first member in the bend region to cover the openings.

The second member also is a tube having rigid proximal and distal ends, and the portion of the second member that lies within the bend region is relieved with a series of axially spaced, circumferentially extending slots to provide the relative flexibility. A motor applies the force as torque to the proximal end of the second member, and the slotted flexible portion is configured to transmit the torque through the bend region to rotate the cutting implement (which is, eg. a blade). In one embodiment, pliable material is disposed in some or all of the slots. The pliable material helps avoid tissue fragments severed by the cutting implement (which, together with irrigation fluid, are removed by suction from the surgical site through the second member) from becoming lodged on the edges of the slots. The pliable material also reduces the axial compressibility of the inner tube and leaks in the suction applied to the proximal end of the inner tube.

Preferably the surgical instrument comprises a first member provided with a bend region that angularly offsets said distal region from said axis in a selected direction,
at least a portion of said second member that is disposed within said bend region being relatively flexible, and
said first member being relatively flexible at least in said bend region to selectively change a rotational orientation of said opening with respect to said axis without changing said selected direction of said offset.

The bend region is provided by a rigid member that is disposed coaxially with the first and second members and is curved in the bend region. The rigid member radially separates the first member from the second member at least in the bend region. This helps avoid interference between the flexible regions (eg. the edges of the slots) as the second member moves. Preferably, the second member is disposed within the rigid member, which is in turn disposed within the first member.

The rigid member has an open distal tip disposed proximally of the cutting implement and opening, and the first and second members are configured to contact each other distally of the tip to maintain the cutting implement in tissue cutting relationship with edges of the opening. A portion of the distal region of the first member has a reduced inner diameter with respect to the remainder of the first member to provide the contact with the second member and abuts the tip of the rigid member. The reduced inner diameter equals the inner diameter of the rigid member to provide a substantially smooth chamber within which the second member rotates, thus reducing the risk of the inner member seizing as it rotates.

The first member is rotatable to allow orientation of the opening to be changed over an arc of at least 180°C, and preferably over a range of 360°C. The outer tube is rotated manually, using a knob that is rigidly secured to a proximal end of the first member and rotatably mounted to a stationary portion of the base. A ratchet mechanism mounts the knob to the base to allow the knob to be selectively rotated to a plurality of discrete positions, thereby to allow the opening for the cutting implement to be selectively positioned to a corresponding plurality of discrete rotational orientations.

Preferably the surgical instrument comprises a stationary support tube that extends distally from a base disposed coaxially between said first and second members.

The stationary portion includes a plurality of recesses each of which corresponds to one of the discrete positons, and the knob has a plunger that selectively engages the recesses to maintain the opening in the discrete rotational orientation that the user has selected. The knob is resiliently biased toward said stationary portion to retain said plunger in a recess. This helps avoid accidental rotation of the first member with respect to the base.

The proximal end of the second member is secured to a drive shaft mounted for movement (eg. rotation) with respect to the stationary portion and the knob. The drive shaft is driven by a motor to rotate the second member with respect to the first member and move the cutting implement. The second member receives suction at its proximal end to draw tissue fragments and other body material cut by the cutting implement thorugh the second member away from a surgical site while the instrument remains in situ for futher cutting.

Other features and advantages of the invention will become apparent from the following detailed description, and from the claims.

The invention will now be described with reference to the accompanying drawings.

Figure 1 shows a surgical instrument according to the invention, having a cutting implement that is adjustable to different rotational positions.

Figure 2 is a partial cross-sectional view of portions of the instrument of Figure 1, showing details of the tip and base.

Figures 3 - 5 show inner, intermediate, and outer tubes, respectively, of the surgical instrument of Figure 1.

Figure 6 is a cross-section of the base of the surgical instrument, taken along line 6-6 of Figure 2.

Figure 7 shows the surgical instrument of Figure 1 in use.

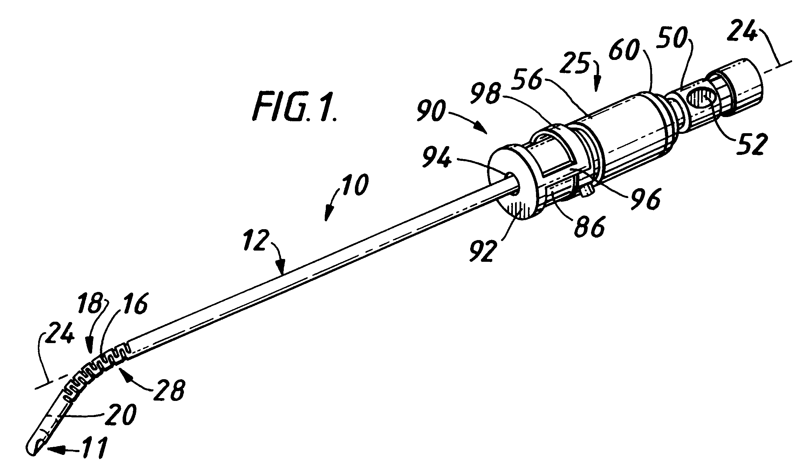

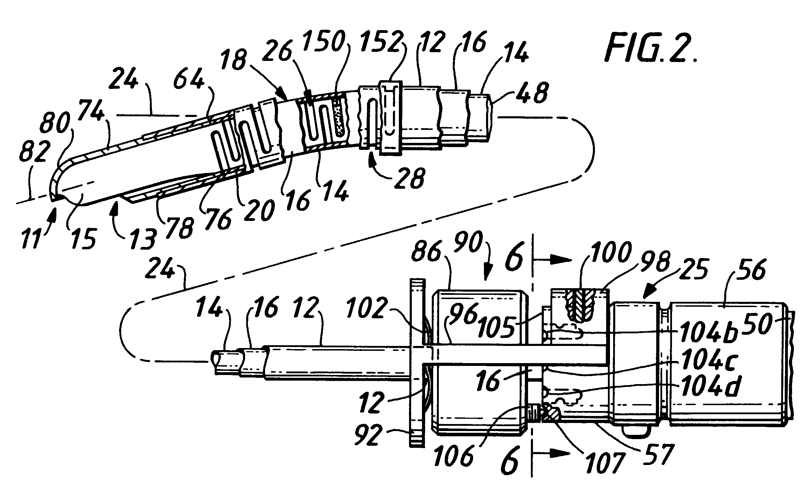

Referring to Figures 1 and 2, surgical instrument 10 suitable for performing, eg. closed, arthroscopy surgery on the knee with a surgical tool 11, includes an outer tube 12 within which a rotating inner tube 14 is coaxially disposed. The distal end of outer tube 12 includes an opening 13, the edges of which are sharpened and serrated, through which a cutting implement 15 (formed by sharpened, serrated edges of a similar opening in the distal end of inner tube 14) of surgical tool 11 is periodically exposed as inner tube 14 rotates. A rigid, stationary intermediate tube 16 is disposed coaxially between outer tube 12 and inner tube 14. Intermediate tube 16 is curved through a bend region 18 disposed slightly proximally of the distal end 20 of tube 16 to angularly offset surgical tool 11 from a generally straight axis 24 of surgical instrument 10. Bend region 18 enables surgical instrument 10 to operate on surgical areas that are difficult to reach with a straight instrument.

Tubes 12, 14 and 16 are proximally supported by a base 25. As discussed below, inner tube 14 includes a slotted, flexible region 26 disposed within bend region 18 to accept the curvature imposed by bend region 18 and transmit torque (and other forces) applied at base 25 through bend region 18 to rotate cutting implement 15 with sufficient force to sever tissue or other body material exposed through opening 13. Outer tube 12 has a slotted, flexible region 28 that envelopes bend region 18 and allows the user to rotate outer tube 12 with respect to base 25, despite the curvature imposed by bend region 18. This feature enables the user to selectively change the rotational orientation of opening 13, and hence surgical tool 11, with respect to axis 24 without rotating the entire surgical instrument 10, and thus without changing the orientation of bend region 18 and the angular offset that it provides. As a result, the user can maintain surgical instrument 10 in an essentially fixed position, while changing the angle of attack of cutting implement 15 by rotating outer tube 12.

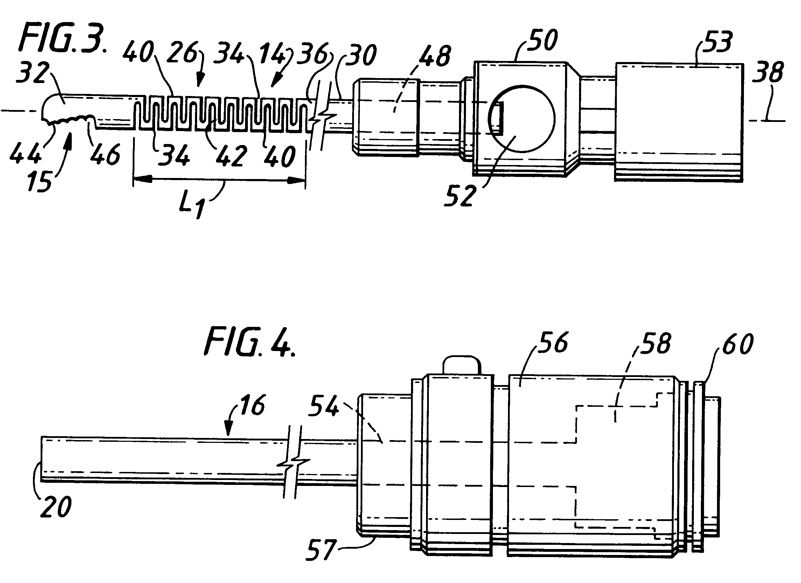

Referring also to Figure 3, inner tube 14 is made from metal, such as stainless steel, and has rigid proximal and distal regions 30, 32, that are connected by flexible region 26. Flexible region 26 is relieved with an axially extending series of circumferential slots 34 disposed in the walls 36 of tube 14 and is continuous with the adjacently disposed proximal and distal regions 30, 32. (Slotting a rotatable tube for flexibility is described in US-A-5 152 744). Slots 34 are perpendicular to the longitudinal axis 38 of tube 14 and are arranged in a symmetrical pattern along the length L₁ of flexible region 26 to provide uniform flexibility as inner tube 14 rotates. This minimizes torsional stresses on inner tube 14 and helps prolong the operating life of surgical instrument 10.

Slots 34 are disposed parallel to each other (vertically in Figure 3) along length L₁. Adjacent slots 34 extend into tube 14 from opposite directions (eg. from above and below tube 14 in Figure 3) and are circumferentially offset from each other by 180°. The number of slots 34, their dimensions (ie. their width and depth), and the spacing between adjacent slots are a function of the desired degree of flexibility. For example, the width of each slot 34 and the spacing between slots 34 and the spacing between slots 34 each are 0.5mm (0.02 inches).

A tab 40 bounds each slot 34 circumferentially, and adjacent tabs 40 are interconnected by annular rings 42, which provide the spacing between adjacent slots 34. The interconnected series of rings 42 and tabs 40 provide a series of interconnected, integrally formed "U" shaped leaf springs along the length L₁ of flexible region that give uniform flexibility and efficiently transmit torque (ie. rotational force) applied at proximal region 30 of tube 14 to distal region 32 through the curvature imposed by bend region 18 (Figure 1). The depth of slots 34 (ie. rotational force) applied at proximal region 30 of tube 14 to distal region 32 through the curvature imposed by bend region 18 (Figure 1). The depth of slots 34 (ie. the amount of which slots 34 extend radially into tube 14) is a function of the desired torsional strength of flexible region 26. For example, slots 34 have a depth of about 75% of the outer diameter (3.4mm, 0.135 inches).

The length L₁ of flexible region 26 is a function of the length of bend region 18. Flexible region 26 should be sufficiently long (eg. 17.6mm, 0.7 inches) so as to span the entire length of bend region 18, with adjacent rigid regions 30, 32 lying within straight regions of stationary intermediate tube 16. This allows flexible region 26 to make a smooth transition between the straight regions of intermediate tube 16 and bend region 18, thereby reducing stresses imposed by the curved inner walls of bend region on walls 36 of inner tube 14.

Flexible region 26 can be formed by any suitable method. Examples include wire EDM (electric discharge machining) and sawing. Both are described in the aforementioned US Patent 5 152 744.

Distal region 32 of inner tube 14 supports cutting implement 15 (which is, for example, stainless steel and attached to tube 14 by welding or brazing). Cutting implement 15 is defined by serrated, sharpened edges 44 of a distal opening 46 in tube 14 and is sized to provide a close running fit with the distal end of outer tube 12 for efficient cutting. Opening 46 is an extension of a central aperture 48 in inner tube that runs the entire length of tube 14.

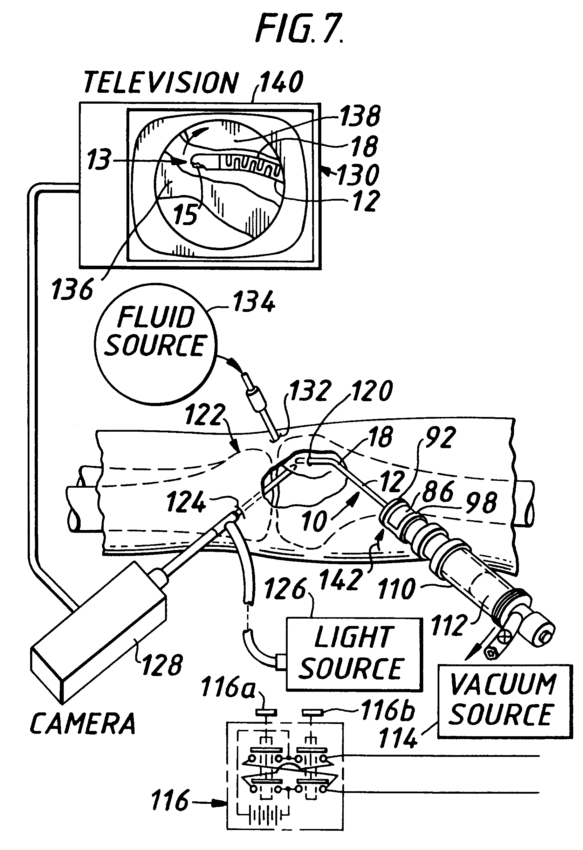

Proximal region 30 of inner tube 14 is rigidly mounted to a drive shaft 50 that rotates within base 25. Central aperture 48 terminates in a vacuum source opening 52 in drive shaft 50. The proximal end 53 of drive shaft 50 fits into a handpiece 110 (Figure 7), which includes a motor 112 for rotating drive shaft 50 and inner tube 14 with resepct to tubes 12, 16. One example of such a handpiece is described in US Patent No.4705038, entitled "Surgical System for Powered Instruments", and assigned to the present assignee, which is incorporated by reference. Opening 52 is coupled to a vacuum source 114 (Figure 7) during operation to remove severed tissue and irrigating fluid from the surgical site via aperture 48 in a manner described in detail below.

Figure 4 shows intermediate tube 16 (before bend region 18 is formed), which is made from a rigid material such as metal (eg. stainless steel). Intermediate tube 16 is hollow along its entire length to provide a passage 54 that receives inner tube 14, which protrudes through the open distal end 20 of intermediate tube 16 is only slightly larger than the outer diameter of inner tube 14 (eg. by approximately 0.05mm (0.002 inches)); this allows inner tube 14 to keep the sharp cutting edges of implement 15 and opening 13 closely aligned.

The proximal end of intermediate tube 16 is rigidly mounted to a hub 56 of base 25. A cavity 58 in hub 56 communicates with passage 54 and is configured to receive drive shaft 50. During assembly, inner tube 14 is inserted through hub 56 into intermediate tube 16 (before bend region 18 if formed). A pliable fitting 60 retains drive shaft 50 within hub 56. Fitting 60 provides a fluid-tight seal when base 25 is inserted into handpiece 110.

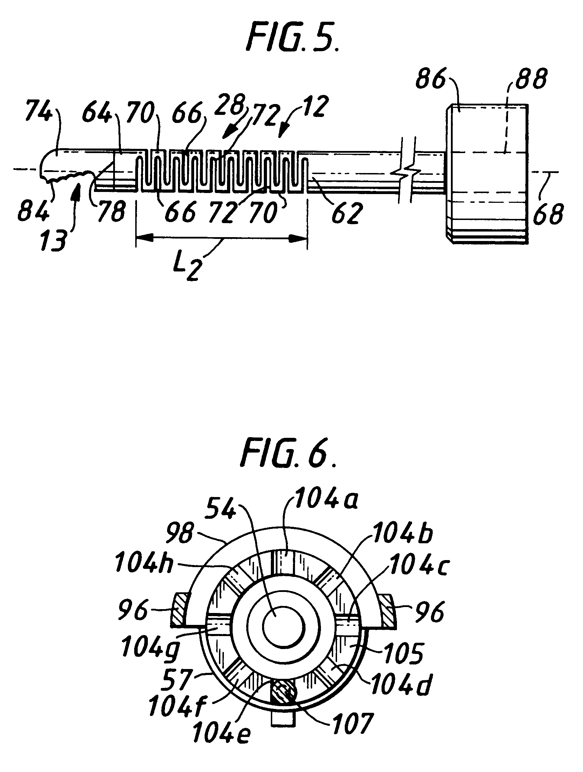

Referring to Figure 5, outer tube 12 is essentially a larger version of inner tube 14 and includes rigid proximal and distal regions 62, 64 that are integrally connected by flexible region 28. Flexible region 28 includes an axially extending series of slots 66 disposed perpendicularly to the longitudinal axis 68 of tube 12 and arranged in a symmetrical pattern along the length L₂ of flexible region 28. Adjacent slots 66 extend radially into tube 12 in opposite directions (ie. from above and below tube 12 in Figure 5). Each slot 66 is approximately 0.63mm (0.025 inches) wide and has a depth of about 3.5mm (0.140 inches).

Each slot 66 is bounded by a tab 70. Adjacent tabs 70 are circumferentially offset by 180° and are connected by rings 72 (each of which has the same width as slots 66) to form a series of "U"shaped springs that are continuous with each other and with proximal and distal regions 62, 64. As a result, flexible region 28 is both sufficiently pliable to accept the curvature imposed by bend region 18 and sufficiently torsionally stiff to transmit applied rotational force through bend region 18 to rotate opening 13. Length L₂ should be such that flexible region 28 spans the entire length of bend region 18, with the adjacently-disposed rigid portions 62, 64 of outer tube being aligned with straight portions of intermediate tube 16.

As shown most clearly in Figure 2, to ensure a close running fit between sharp edges 44 of cutting implement 15 and the corresponding cutting edges 84 of opening 13 despite the spacing between tubes 12, 14 that intermediate tube 16 provides, a distal extension 74 having the same inner diameter as intermediate tube 16 is secured to outer tube 12 at distal end 64. Extension 74 is, eg. stainless steel and is welded or brazed to outer tube 12, which can be a softer material, such as aluminum. The proximal end of extension 74 has a reduced outer diameter to allow it to be disposed within outer tube 12 and about intermediate tube 16 at joint 76. A shoulder 78 on distal extension 74 limits the amount by which extension 74 is inserted into distal end 64 during assembly.

Opening 13 is disposed in a distal tip 80 of extension 74 and faces somewhat to the side of outer tube 12. That is, opening 13 does not extend completely to the centreline 82 of extension 74. As a result, while surgical tool will cut tissue that enters opening 13 from the distal end of instrument 10, the majority of the cutting action is to one side. Moreover, tip 80 provides distal support for the rotating inner tube 14. The edges 84 of opening 13 are sharpened and serrated to cooperate with sharp edges 44 of cutting implement 15. The clearance between inner tube 14 and the inner diameter of outer tube extension 74 and intermediate tube 16 is small (eg. approximately 0.05mm (0.002 inches)) to maintain the close running fit between edges 44, 84 while allowing inner tube 14 to rotate freely. The identical inner diameters of extension 74 and intermediate tube 16 avoid inner tube 14 scoring or seizing as it rotates.

Proximal region 62 (Figure 5) of outer tube 12 is rigidly secured to a drum 86 at a sealed joint. Drum 86 serves as a knob to enable the user to manually rotate tube 14, and is rotatably mounted to base 25 in a manner described below. A central passage 88 extends through outer tube 12 and drum 86 to receive intermediate tube 16 and inner tube 14. The inner diameter of outer tube 12 (proximally of extension 74) only slightly exceeds the outer diameter of intermediate tube 16 (eg. by approximately 0.05 mm (0.002 inches)). This allows the user to rotate outer tube 12 but avoids excessive play between tubes 12, 16.

Referring to Figure 2, outer tube 12 and drum 86 are rotatably mounted to base 25 with a spring-loaded rotation assembly 90. Drum 86 is captured between the distal end 57 of hub 56 and a faceplate 92, which includes an opening 94 (Figure 1) through which outer tube 12 projects. A pair of axially extending bars 96 connect faceplate 92 to a sleeve 98 that is rigidly mounted to a hub distal end 57 by one or more press-fit pins 100. A spring 102 (eg. a wave washer), which fits within a recess (not shown) in faceplate 92, resiliently biases drum 86 toward hub 56.

Referring also to Figure 6 (which, for clarity does not show tubes 14, 16 in cross-section), distal end 57 of hub 56 includes a series of (such as eight) rounded recesses 104a-104h disposed in an annular surface 105 of hub 56 that faces drum 86. Recesses 104a-104h are spaced by equal amounts (such as by 45°) around the circumference of hub 56. Surface 105 is flat between adjacent recesses 104a-104h. A plunger 106 having a spring-loaded, ball shaped tip 107 is threaded into drum 86. Tip 107 is resiliently urged against hub 56 and into a selected one of recesses 104a-104h by spring 102.

Thus, the user can selectively rotate drum 86 _ and hence outer tube 12 and surgical tool opening 13 _ to one of eight discrete rotational orientations. The biasing provided by spring 102 maintains plunger tip 107 in the selected recess 104a-104h to avoid accidental rotation. As drum 86 is rotated between recesses 104a-104h, tip 107 is compressed into plunger 106 by flat surfaces 105. Recesses 104a-104h are arranged to allow opening 13 to be rotated in a ratchet-like fashion to commonly used positions with respect to axis 24. For example, positioning plunger tip 107 in recess 104a orients opening 13 oppositely from the direction of curvature of bend region 18 (Figure 2), that is, is aligned with the curvature direction and is oriented downwardly (the position shown in Figure 2). Similarly, recesses 104c and 104g correspond to left and right orientations. Recesses 104b, 104d, 104f and 104h provide intermediate positions for opening 13.

Referring also to Figure 7, in operation, surgical instrument 10 is inserted into the distal end of a handpiece 110 and is introduced as shown through a puncture wound 120 into the knee joint 122, below the patella. Light is projected into the joint via a second puncture 124 using a fibre optic light source 126, and a visual image of the surgical site is returned through a separate optical path to a television camera 128. The image is delivered by camera 128 onto a television screen 130 for viewing by the surgeon. (Alternatively, the surgeon can view the image using an eyepiece, or the image can be recorded).

The surgeon operates surgical tool 11 by activating motor 112, which receives operating potential and current from power supply 116. Motor 112 engages and rotates drive shaft 50, thereby applying rotational force to inner tube 14 and rotating tube 14 with respect to tubes 12, 16. The surgeon controls rotational speed and direction (either unidirectional or oscillatory) using foot switches 116a, 116b, which control the magnitude and polarity of operating potential and current provided by power supply 116 to motor 112. Motor 112 is capable of rotating inner tube 14 over a wide range of speeds, eg. between about 100 rpm and 5000 rpm, and can deliver a torque of up to 25 oz inches.

Different types of surgical instruments such as instrument 10 have rotational and torsional limits. To prevent the surgeon from inadvertently operating instrument 10 at dangerously high speeds and torques, instrument 10 identifies to sensors (not shown) in handpiece 110 what type of instrument it is, and the spped of and torsion applied by motor 112 is controlled so that these limits are not exceeded. (This control technique is described in the aforementioned US Patent No.4705038.

The torsion that motor 112 provides is efficiently delivered to cutting implement 15 by flexible region 26. Although region 26 is sufficiently flexible to accept the curvature imposed by bend region 18, it has a high degree of torsional stiffness and thus provides good torque response. That is, torsion applied by motor 112 is transmitted to distal region 32 of inner tube 14 substantially immediately when inner tube 14 is rotated from its rest position, without requiring any significant "preloading" of flexible region 26 prior to passing the torque to distal end 32. Also, flexible region 26 does not expand in diameter by any significant amount as it rotates and applies torque to distal end 32, reducing the possibility that tube 14 will bind within intermediate tube 16 during rotation.

During the surgical procedure, the body joint is distended with fluid introduced through a third puncture wound 132 from a fluid source 134. The fluid irrigates the site and renders tissue 136 (which is, eg. synovial tissue) mobile so that it floats and can be displaced (similar to the movement of seaweed in water). Note that synovial tissue 136 is located beneath outer tube 12; thus, drum 86 is positioned so that plunger 106 is in recess 104e (Figures 2 and 6). The curvature provided by bend region 18 allows surgical instrument 10 to be easily positioned to place surgical tool 11 against tissue 136 (even if tissue 136 is located in a region of the joint that cannot easily be reached by a straight instrument) without manipulating instrument 10 unduly or requiring that additional punctures be made to gain access to tissue 136. This reduces patient discomfort, as well as the chances for infection and other deleterious consequences of the surgery.

The surgeon progressively cuts away synovial tissue 136 by moving surgical instrument 10 from side to side and in the axial direction using handpiece 110 (while viewing television screen 130). If during the procedure the surgeon wishes to cut tissue from another region of the synovial tissue, such as region 138 located above outer tube 14, the present invention allows him to do so simply by changing the rotational orientation of surgical tool opening 13 (eg. in the direction of arrow 140) while maintaining handpiece 110 in a fixed position _ that is, without requiring the surgeon to rotate or pivot handpiece 110.

This is accomplished, for example, by grasping drum 86 with the finger and thumb of one hand (while the other hand continues to grasp the body of handpiece 110) and turning drum 86 in the direction in which opening 13 is selected to rotate (eg. along arrow 142). The rotational force applied by the surgeon is transmitted through bend region 18 by flexible region 28, thereby causing distal extension 74 of outer tube 12 to rotate with respect to intermediate tube 16 and base 25 and change the orientation of opening 13 with respect to axis 24 (in this case, by 180°).

In this example, in which tissue 138 is located above outer tuve 12, the surgeon continues to rotate drum 86 until plunger 106 rests within recess 104a. As drum 86 is rotated between recesses, plunger 106 slides across flat surface 105 and drum 86 compresses spring 102 against faceplate 94. Thus, spring 102 positively urges plunger 106 into each recess 104 as it is encountered, thereby giving the surgeon kinesthetic feedback as to the amount by which opening 13 has been rotated.

The surgeon can change the rotational orientation of opening 13 at any time. For example, inner tube 14 can be driven by motor 112 or may be stationary while the surgeon rotates opening 13. Distal extension 74 rotates smoothly with respect to the stationary intermediate tube 16 at joint 76, while providing constant distal support (at tip 80) for rotating inner tube 14. The identical inner diameters of tube 16 and extension 74 help ensure that the rotation of outer tube 12 does not cause inner tube 14 to bind or seize. The surgeon can return to cutting tissue 136 at any time simply by rotating drum 86, either in the opposite direction from arrow 142 or in the same direction to trace a 360° arc from his starting point.

Tissue fragments and other body material cut by surgical tool 11 are withdrawn from the surgical site along with irrigation fluid via central aperture 48 of inner tube 14 (Figure 2) in response to suction applied by vacuum source 114. Note that as flexible region 26 rotates within the bend region 18, the width of each slot 34 at the periphery of tube wall 36 progressively increases and decreases incrementally with respect to its nominal width. This is because flexible region 26 tends to stretch at the apex of bend region 18 (ie. the upper part of bend region 18 in Figure 2) and compress at the base of the bend. This alternating widening and constricting as tube 14 rotates may generate turbulence in the fluid being withdrawn through aperture 48, thereby assisting in the transport of tissue fragments through the chamber and out of surgical instrument 10.

The exposure of aperture 48 to the interior walls of intermediate tube 16 through slots 34 has not been found to allow tissue fragments to become caught in the slots and cause blockage, perhaps due to the small width of the slots and the continual rotation of inner tube 14. Fluid likewise has not been found to seep between tubes 14, 16 via slots 34 (or between tubes 12, 16) in amounts that interfere with the operation of instrument 10.

Other embodiments are within the scope of the following claims.

For example, although surgical instrument 10 is shown with bend region 18 orientated downwardly with respect to axis 24 and handpiece 25, it is readily apparent that other orientations (eg. downwardly, to the right or left, or anywhere in between these directions) are possible. Indeed, a set of surgical instruments may be provided, each with a different bend region 18 orientation, to give the user maximum flexibility in determining the optimum bend configuration for a given surgical procedure other amounts of curvature can be provided.

Also, as described in the aforementioned patent US-A-5 152 744, pliable material (such as silicone rubber) may be disposed in slots 34 of inner tube 14. (Pliable material is illustrated in Figure 2 by shaded area 150 within a slot 34 of inner tube 14). The pliable material would further help avoid clogging by reducing the tendency of tissue fragments to become caught on the edges of slots 34 as the fragments pass through inner tube 14. Moreover, the pliable material is less compressible than empty space, and thus would serve to reduce the axial compressibility of flexible region 26.

A tube (made from, eg. shrink wrap plastic) may be placed over outer tube in bend region 18 to cover slots 66.

(A portion of such a tube 152 is shown in Figure 2). Among other advantages, a shrink wrap tube will avoid material becoming lodged within slots 66 and help prevent the edges of slots 66 (which may be sharp) from causing damage.

Surgical tools other than the cutting implement shown in the figures can be used. For example, surgical tool 11 need not have serrated edges and may alternatively be constructed as a bone abrading instrument. The surgical instrument can be constructed to perform procedures other than arthroscopy (such as laparoscopy).

Inner tube 14 may alternatively be flexible along its entire length so long as the tube is sufficiently stiff to transmit the forces applied to it (eg. torsion) to surgical tool 11. For example, inner tubes 14 may comprise a nonmetal, such as plastic, and drive a separate, metal member that carries cutting implement 15. Such a configuration is shown in EP-A-92907264.3).

While the invention has been described in terms of surgical instruments for arthroscopy, the invention may also be used with other types of instruments, for example, instruments configured for other kinds of endoscopic procedures and for biopsy applications.

## Claims

1. A surgical instrument (10) disposed generally along an axis, said surgical instrument comprising;
a first member (12) that extends distally from a base (25) and has an opening (13) in a distal region thereof for admitting tissue;
a second member (14) disposed within said first member (12) comprising a distal region that rotates in response to force applied to a proximal region (30) of said second member (14)
characterised in that said instrument (10) is provided with actuating means (86) disposed at a proximal region (62) of said first member to selectively change a rotational orientation of said first member with respect to said base (25).

2. The instrument of claim 1 further comprising means for providing said first member (12) with a bend region (18) that angularly offsets said distal region from said axis in a selected direction
said first member (12) being relatively flexible at least in said bend region (18) to allow said actuating means (86) to change the rotational orientation of said opening (13) without changing said selected direction of said offset, and
at least a portion of said second member (14) that is disposed in said bend region (18) being relatively flexible to transmit said applied force through said bend region to said cutting implement (15).

3. The instrument of claim 2 wherein said first member (12) is relieved in said bend region (18) to provide the relative flexibility.

4. The instrument of claim 2 wherein said proximal region and said distal region of said first member (12) are rigid, said first member (12) being relieved to provide the relative flexibility.

5. The instrument of claim 3 or 4 wherein said first member (12) is relieved with plurality of discrete openings disposed in walls thereof.

6. The instrument of claim 5 wherein said openings comprise a series of axially spaced, circumferentially extending slots (66).

7. The instrument of claim 6, wherein said slots extend radially into said first member (12) over approximately 75% of the diameter of said first member.

8. The instrument of claim 6 wherein adjacent ones of said slots (66) extend into said first member from opposite directions.

9. The instrument of claim 2 wherein said proximal end and said distal end of said second member (14) are rigid, said portion of said second member (14) that is disposed in said bend region relieved to provide the relative flexibility.

10. The instrument of claim 9 wherein said portion of said second member (14) is relieved with a series of axially spaced, circumferentially extending slots (34).

11. The instrument of claim 10 further comprising pliable material disposed in at least some of said slots (34).

12. The instrument of claim 9 wherein said force is applied by a motor that applies torque to said proximal end of said second member (14) said relieved portion of said second member (14) being configured to transmit said torque through said bend region to said cutting implement.

13. The instrument of claim 2 wherein said means for providing said bend region includes a rigid member (16) that is disposed coaxially with said first member (12) and said second member (14) and is curved in said bend region.

14. The instrument of claim 13 wherein said first member (12) and said second member (14) are each relieved with a plurality of openings in said bend region to provide said flexibility and said second member (14) moves with respect to said first member (12) to move said cutting implement (15), said rigid member (16) radially separating said first member and said second member at least in said bend region to avoid interference therebetween as said second member moves.

15. The instrument of claim 13 wherein said second member (14) is disposed within said rigid member and said rigid member (16) is disposed within said first member (12).

16. The instrument of claim 15 wherein said rigid member (16) has an open distal tip disposed proximally of said cutting implement (15) and opening, said first member (12) and said second member (14) being configured to contact each other distally of said tip to maintain said cutting implement (15) in tissue cutting relationship with edges of said opening.

17. The instrument of claim 16 wherein a portion of said distal region of said first member (12) has a reduced inner diameter relative to other portions of said first member to provide contact with said second member.

18. The instrument of claim 17 wherein said portion of said distal region of said first member (12) abuts said tip of said rigid member, and said inner diameter is the same as an inner diameter of said rigid member.

19. The instrument of claim 15 wherein at least said first member (12) is relieved with a plurality of openings (66) in said bend region to provide said flexibility, and further comprising a pliable sheath disposed over said first member in said bend region that covers said openings.

20. The instrument of claim 1 wherein said means for rotating allows said rotational orientation of said opening to be changed over a range of at least 180°.

21. The instrument of claim 20 wherein said means for rotating allows said rotational orientation of said opening to be changed over a range of 360°.

22. The instrument of claim 1 wherein said means for rotating is configured to be manually actuated by a user of said instrument to change said rotational orientation of said opening.

23. The instrument of claim 22 wherein said means for rotating includes a knob (86) that is rigidly secured to a proximal end of said first member (12) and rotatably mounted to a stationary portion of said base.

24. The instrument of claim 23 wherein said knob is mounted to said stationary portion so that said knob (86) can be selectively rotated to a plurality of discrete positions, thereby to allow said opening (11) to be selectively positioned to a corresponding plurality of discrete rotational orientations.

25. The instrument of claim 24 wherein said stationary portion includes a plurality of recesses each of which corresponds to one of said discrete positions, said knob (86) including a plunger (106) for selectively engaging said recesses (104), to maintain said opening in the corresponding discrete rotational orientation.

26. The instrument of claim 25 wherein said knob is resiliently biased toward said stationary portion to retain said plunger (106) in a said recess (104), thereby to avoid accidental rotation of said first member with respect to said base.

27. The instrument of claim 23 wherein said proximal end of said second member (14) is secured to a drive shaft mounted for movement with respect to said stationary portion and said knob.

28. The instrument of claim 27 wherein said drive shaft is adapted to be driven by a motor to rotate said second member (14) with respect to said first member (12) and move said cutting implement (15).

29. The instrument of claim 1 wherein said second member (14) is hollow and is adapted to receive suction at its proximal end and to transport body material cut by said cutting implement away from a surgical site while the instrument remains in situ for further cutting.

30. The instrument of claim 1 wherein said cutting implement (15) comprises a blade.

31. The instrument of claim 1 wherein said first member (12) is provided with a bend region that angularly offsets said distal region from said axis in a selected direction;
at least a portion of said second member (14) that is disposed within said bend region being relatively flexible;
said first member (12) being relatively flexible at least in said bend region to selectively change a rotational orientation of said opening with respect to said axis without changing said selected direction of said offset.

32. The instrument of claim 31 wherein said first member is relieved in said bend region with a plurality of discrete openings (66) disposed in walls thereof to provide the relative flexibility.

33. The instrument of claim 31 wherein said portion of said second member is relieved with a plurality of discrete openings (34) disposed in walls thereof to provide the relative flexibility.

34. The instrument of claim 32 wherein said force is applied by a motor that applies torque to proximal end of said second member (14), said portion of said second member (14) being configured to transmit said torque through said bend region to said cutting implement (15).

35. The instrument of claim 31 wherein said means for providing said bend region includes a rigid member (16) that is disposed coaxially with said first member (12) and said second member (14) and is curved in said bend region.

36. The instrument of claim 35 wherein said first member (12) and said second member (14) are each relieved with a plurality of openings (66, 34) in said bend region to provide said flexibility and said second member rotates with respect to said first member to move said cutting implement, said rigid member (16) radially separating said first member and said second member at least in said bend region to avoid interference therebetween as said second member rotates.

37. The instrument of claim 35 wherein said second member (14) is disposed within said rigid member (16) and said rigid member is disposed within said first member (14).

38. The instrument of claim 31 wherein said means for rotating is configured to be manually actuated by a user of said instrument to change said rotational orientation of said opening.

39. The instrument of claim 38 wherein said means for rotating includes a knob (86) that is rigidly secured to a proximal end of said first member (12) and rotatably mounted to a stationary portion of said base.

40. The instrument of claim 39 wherein said knob (86) is mounted to said stationary portion so that said knob can be selectively rotated to a plurality of discrete positions, thereby to allow said opening to be selectively positioned to a corresponding plurality of discrete rotational orientations.

41. The surgical instrument of claim 1 or 2 wherein said instrument further comprises;
a stationary support tube (16) that extends distally from a base disposed coaxially between said first and second members (12, 14).

42. The instrument of claim 41 wherein said first and second members (12, 14) and said support tube (16) are each relieved in said bend region to provide the relative flexibility.

43. The instrument of claim 42 wherein said first member (12) and said second member (14) are each relieved with a plurality of discrete openings (66, 34) disposed in walls thereof.

44. The instrument of claim 41 wherein said force is applied by a motor that applied torque to said proximal end of said second member (14), said portion of said second member (14) being relieved to provide the relative flexibility and being configured to transmit said torque through said bend region to said cutting implement.

45. The instrument of claim 41 wherein said support tube (16) has an open distal tip disposed proximally of said cutting implement and opening, said first member and said second member being configured contact each other distally of said tip to maintain said cutting implement (15) in tissue cutting relationship with edges of said opening.

46. The instrument of claim 45 wherein a portion of said distal region of said first member (12) has a reduced inner diameter relative to other portions of said first member to provide said contact with said second member (14).

47. The instrument of claim 41 wherein said actuating means (86) is configured to be manually actuated by a user of said instrument to change said rotational orientation of said opening.

48. The instrument of claim 47 wherein said actuating means includes a knob (86) that is rigidly secured to a proximal end of said first member (12) and rotatably mounted to a stationary portion of said base.

49. The instrument of claim 48 wherein said knob (86) is mounted to said stationary portion so that said knob (86) can be selectively rotated to a plurality of discrete positions, thereby to allow said opening to be selectively positioned to a corresponding plurality of discrete rotational orientations.

## Patentansprüche

1. Chirurgisches Instrument (10), welches generell entlang einer Achse angeordnet ist mit
einem ersten Element (12), welches sich distal von einer Halterung (25) erstreckt und in einem distalen Bereich desselben eine Öffnung (13) zum Aufnehmen von Gewebe aufweist,
einem zweiten Element (14), welches innerhalb des ersten Elements angeordnet ist und einen distalen Bereich aufweist, der unter der Einwirkung einer Kraft rotiert, die auf einen proximalen Bereich (30) des zweiten Elementes (14) aufgebracht wird,
**dadurch gekennzeichnet**, daß das Instrument (10) mit einem Betätigungsmittel (86) versehen ist, das an einem proximalen Bereich des ersten Elementes angeordnet ist, um eine Rotationsorientierung des ersten Elementes in bezug auf die Halterung (25) wahlweise zu ändern.

2. Instrument nach Anspruch 1, welches weiterhin Mittel aufweist, um das erste Element (12) mit einem gebogenen Bereich (18) zu versehen, welcher den distalen Bereich gegenüber der Achse in einer gewählten Richtung winkelmäßig versetzt,
wobei das erste Element (12) zumindest in dem gebogenen Bereich (18) relativ flexibel ist, um zu ermöglichen, daß das Betätigungsmittel (86) die Rotationsorientierung der Öffnung (13) ändert, ohne die gewählte Richtung der Versetzung zu ändern, und
wenigstens ein Abschnitt des zweiten Elementes (14), der in dem gebogenen Bereich (18) angeordnet ist, relativ flexibel ist, um die aufgebrachte Kraft durch diesen gebogenen Bereich auf das Schneidwerkzeug (15) zu übertragen.

3. Instrument nach Anspruch 2, bei welchem das erste Element (12) in dem gebogenen Bereich reduziert ist, um die relative Flexibilität zu bewirken.

4. Instrument nach Anspruch 2, bei welchem der proximale Bereich und der distale Bereiche des ersten Elementes (12) starr sind und das erste Element (12) reduziert ist, um die relative Flexibilität zu bewirken.

5. Instrument nach Anspruch 3 oder 4, bei welchem das erste Element (12) durch mehrere diskrete Öffnungen, die in den Wandungen desselben angeordnet sind, reduziert ist.

6. Instrument nach Anspruch 5, bei welchem die Öffnungen eine Reihe von sich in Umfangsrichtung erstreckenden Schlitzen (66) aufweisen, die in axialen Abständen voneinander angeordnet sind.

7. Instrument nach Anspruch 6, bei welchem die Schlitze sich radial in das erste Element über etwa 75% des Durchmesser des ersten Elementes erstrecken.

8. Instrument nach Anspruch 6, bei welchem benachbarte Schlitze (66) sich von entgegengesetzten Richtungen in das erste Element erstrecken.

9. Instrument nach Anspruch 2, bei welchem das proximale Ende und das distale Ende des zweiten Elementes (14) starr sind und der Abschnitt des zweiten Elementes (14), der in dem gebogenen Bereich angeordnet ist, reduziert ist, um die relative Flexibilität zu bewirken.

10. Instrument nach Anspruch 9, bei welchem der Abschnitt des zweiten Elementes (14) durch eine Reihe sich in Umfangsrichtung erstreckender Schlitze (34) reduziert ist, die einen axialen Abstand voneinander aufweisen.

11. Instrument nach Anspruch 10, das weiterhin biegsames Material aufweist, das in wenigstens einigen der Schlitze (34) angeordnet ist.

12. Instrument nach Anspruch 9, bei welchem die Kraft durch einen Motor aufgebracht wird, der ein Drehmoment auf das proximale Ende des zweiten Elementes (14) aufbringt, und der reduzierte Abschnitt des zweiten Elementes (14) so ausgebildet ist, daß das Drehmoment durch den gebogenen Bereich zum Schneidwerkzeug übertragen wird.

13. Instrument nach Anspruch 2, bei welchem das Mittel zum Bereitstellen des gebogenen Bereiches ein starres Element (16) aufweist, welches koaxial zum ersten Element (12) und dem zweiten Element angeordnet ist und in dem gebogenen Bereich gekrümmt ist.

14. Instrument nach Anspruch 13, bei welchem das erste Element (12) und das zweite Element (14) jeweils durch mehrere Öffnungen in dem gebogenen Bereich reduziert sind, um die Flexibilität zu bewirken, und das zweite Element (14) in bezug auf das erste Element (12) sich bewegt, um das Schneidwerkzeug (15) zu bewegen, und das starre Element (16) das erste Element und das zweite Element wenigstens in dem gebogenen Bereich radial trennt, um zu vermeiden, daß diese beiden Elemente einander stören, wenn das zweite Element sich bewegt.

15. Instrument nach Anspruch 13, bei welchem das zweite Element innerhalb des starren Elementes angeordnet ist und das starre Element (16) innerhalb des ersten Elementes (12) angeordnet ist.

16. Instrument nach Anspruch 15, bei welchem das starre Element (16) ein offenes distales Ende hat, welches proximal zum Schneidwerkzeug (15) und zur Öffnung angeordnet ist, und erstes Element (12) und zweites Element (14) so ausgebildet sind, daß sie einander distal von diesem Ende berühren, um das Schneidwerkzeug (15) in gewebeschneidender Relation zu Kanten der Öffnung zu halten.

17. Instrument nach Anspruch 16, bei welchem ein Abschnitt des distalen Bereiches des ersten Elementes (12) einen verringerten inneren Durchmesser relativ zu anderen Abschnitten des ersten Elementes aufweist, um eine Berührung mit dem zweiten Element zu bewirken.

18. Instrument nach Anspruch 17, bei welchem dieser Abschnitt dieses distalen Bereiches des ersten Elementes (12) an dem Ende des starren Elementes anstößt und der innere Durchmesser der gleiche ist wie ein innerer Durchmesser des starren Elementes.

19. Instrument nach Anspruch 15, bei welchem wenigstens das erste Element (12) mittels mehrerer Öffnungen (22) in dem gebogenen Bereich reduziert ist, um die Flexibilität zu bewirken, und weiterhin eine biegsame Umhüllung auf dem ersten Element in dem gebogenen Bereich angeordnet ist, welches die Öffnungen abdeckt.

20. Instrument nach Anspruch 1, bei welchem das Mittel zum Rotieren eine Änderung der Rotationsorientierung der Öffnung über einen Bereich von wenigstens 180° ermöglicht.

21. Instrument nach Anspruch 20, bei welchem das Mittel zum Rotieren eine Änderung der Rotationsorientierung der Öffnung über einen Bereich von 360° ermöglicht.

22. Instrument nach Anspruch 1, bei welchem das Mittel zum Rotieren so ausgebildet ist, daß es durch einen Benutzer des Instrumentes manuell betätigbar ist, um die Rotationsorientierung der Öffnung zu ändern.

23. Instrument nach Anspruch 22, bei welchem das Mittel zum Rotieren einen Griffknopf (86) aufweist, der starr an einem proximalen Ende des ersten Elements (12) befestigt und rotierbar an einen stationären Teil der Halterung angebracht ist.

24. Instrument nach Anspruch 23, bei welchem der Griffknopf so an dem stationären Teil angebracht ist, daß der Griffknopf (86) wahlweise in mehrere diskrete Positionen rotiert werden kann, um dadurch die wahlweise Positionierung der Öffnung (11) in einer entsprechenden Anzahl diskreter Rotationsorientierungen zu ermöglichen.

25. Instrument nach Anspruch 24, bei welchem der stationäre Teil mehrere Ausnehmungen aufweist, von denen jeder einer der diskreten Positionen entspricht, und der Griffknopf (86) einen Raststift (106) zum wahlweisen Eingriff in die Ausnehmungen (104) aufweist, um die Öffnung in der entsprechenden diskreten Rotationsorientierung zu halten.

26. Instrument nach Anspruch 25, bei welchem der Griffknopf elastisch in Richtung auf den stationären Teil beaufschlagt ist, um den Raststift (106) in einer der Ausnehmungen (104) zu halten und dadurch eine unbeabsichtigte Rotation des ersten Elementes in bezug auf die Halterung zu vermeiden.

27. Instrument nach Anspruch 23, bei welchem das proximale Ende des zweiten Elementes (14) an einer Antriebswelle befestigt ist, die gegenüber dem stationären Teil und dem Griffknopf bewegbar gelagert ist.

28. Instrument nach Anspruch 27, bei welchem die Antriebswelle so ausgebildet ist, daß sie durch einen Motor antreibbar ist, um das zweite Element (14) in bezug auf das erste Element (12) zu rotieren und das Schneidwerkzeug (15) zu bewegen.

29. Instrument nach Anspruch 1, bei welchem das zweite Element (14) hohl und so ausgeführt ist, daß es an seinem proximalen Ende einer Saugwirkung unterliegen kann und Körpermaterial, welches durch das Schneidwerkzeug abgeschnitten worden ist, von einer Operationsstelle wegtransportieren kann, während das Instrument für weitere Schneidvorgänge an Ort und Stelle bleibt.

30. Instrument nach Ansrpuch 1, bei welchem das Schneidwerkzeug (15) eine Klinge aufweist.

31. Instrument nach Anspruch 1, bei welchem das erste Element (12) mit einem gebogenen Bereich versehen ist, der den distalen Bereich gegenüber der Achse in einer gewünschten Richtung winkelmäßig versetzt,
wobei wenigstens ein Abschnitt des zweiten Elementes (14), der innerhalb des gebogenen Bereiches angeordnet ist, relativ flexibel ist und
das erste Element (12) wenigstens in dem gebogenen Bereich relativ flexibel ist, um wahlweise eine Rotationsorientierung der Öffnung in bezug auf die Achse zu ändern, ohne die gewählte Richtung der Versetzung zu ändern.

32. Instrument nach Anspruch 31, bei welchem das erste Element in dem gebogenen Bereich durch mehrere diskrete Öffnungen (66) reduziert ist, die in den Wandungen desselben angeordnet sind, um die relative Flexibilität zu bewirken.

33. Instrument nach Anspruch 31, bei welchem der Abschnitt des zweiten Elementes mittels mehrerer in den Wandungen desselben angeordneter diskreter Öffnungen (34) reduziert ist, um die relative Flexibilität zu bewirken.

34. Instrument nach Anspruch 32, bei welchem die Kraft durch einen Motor aufgebracht wird, welcher ein Drehmoment auf dem proximalen Ende des zweiten Elementes (14) aufbringt, und der Abschnitt des zweiten Elementes (14) so ausgebildet ist, daß er das Drehmoment durch den gebogenen Bereich zum Schneidwerkzeug (15) überträgt.

35. Instrument nach Anspruch 31, bei welchem das Mittel zum Bereitstellen des gebogenen Bereiches ein starres Element (16) aufweist, welches koaxial zum ersten Element (12) und zum zweiten Element (14) angeordnet ist und in dem gebogenen Bereich gekrümmt ist.

36. Instrument nach Anspruch 35, bei welchem das erste Element (12) und das zweite Element (14) jeweils durch mehrere Öffnungen (66, 34) in dem gebogenen Bereich reduziert sind, um die Flexibilität zu bewirken, und das zweite Element in bezug auf das erste Element rotiert, um das Schneidwerkzeug zu bewegen, und das starre Element (16) das erste Element und das zweite Element wenigstens in dem gebogenen Bereich radial trennt, um zu vermeiden, daß die beiden Elemente einander stören, wenn das zweite Element rotiert.

37. Instrument nach Anspruch 35, bei welchem das zweite Element (14) innerhalb des starren Elementes (16) angeordnet ist und das starre Element innerhalb des ersten Elementes (14) angeordnet ist.

38. Instrument nach Anspruch 31, bei welchem das Mittel zum Rotieren so ausgebildet ist, daß es manuell durch einen Benutzer des Instrumentes betätigbar ist, um die Rotationsorientierung der Öffnung zu ändern.

39. Instrument nach Anspruch 38, bei welchem das Mittel zum Rotieren einen Griffknopf (86) aufweist, welcher starr am proximalen Ende des ersten Elementes (12) befestigt und rotierbar an einem stationären Teil der Halterung angebracht ist.

40. Instrument nach Anspruch 39, bei welchem der Griffknopf (86) an dem stationären Abschnitt derart angebracht ist, daß der Griffknopf wahlweise in mehrere diskrete Positionen rotierbar ist, um dadurch eine wahlweise Positionierung der Öffnung in einer entsprechenden Anzahl diskreter Rotationsorientierungen zu ermöglichen.

41. Chirurgisches Instrument nach Anspruch 1 oder 2, bei welchem das Instrument weiterhin versehen ist mit
einem stationären Stützrohr (16), welches sich distal von einer Halterung erstreckt und koaxial zwischen erstem und zweiten Element (12, 14) angeordnet ist.

42. Instrument nach Anspruch 41, bei welchem erstes und zweites Element (12, 14) und das Stützrohr (16) in dem gebogenen Bereich jeweils reduziert sind, um die relative Flexibilität zu bewirken.

43. Instrument nach Anspruch 42, bei welchem das erste Element (12) und das zweite Element (14) jeweils durch mehrere diskrete Öffnungen (66, 34) reduziert sind, die in deren Wandungen angeordnet sind.

44. Instrument nach Anspruch 41, bei welchem die Kraft durch einen Motor aufgebracht wird, welcher Drehmoment auf das proximale Ende des zweiten Elementes (14) aufbringt, und der Abschnitt des zweiten Elementes (14) reduziert ist, um die relative Flexibilität zu bewirken, und ausgebildet ist, um das Drehmoment durch den gebogenen Bereich auf das Schneidwerkzeug zu übertragen.

45. Instrument nach Anspruch 41, bei welchem das Stützrohr (16) ein offenes distales Ende hat, welches proximal zum Schneidwerkzeug und zur Öffnung angeordnet ist, und das erste Element und das zweite Element so ausgebilde t sind, daß sie einander distal zu dem Ende berühren, um das Schneidwerkzeug (15) in einer gewebeschneidenden Relation zu den Kanten der Öffnung zu halten.

46. Instrument nach Anspruch 45, bei welchem ein Abschnitt des distalen Bereiches des ersten Elementes (12) einen kleineren inneren Durchmesser verglichen mit anderen Abschnitten des ersten Elementes aufweist, um die Berührung mit dem zweiten Element (14) zu bewirken.

47. Instrument nach Anspruch 41, bei welchem das Betätigungsmittel (86) so ausgebildet ist, daß es manuell durch einen Benutzer des Instrumentes betätigbar ist, um die Rotationsorientierung der Öffnung zu ändern.

48. Instrument nach Anspruch 47, bei welchem das Betätigungsmittel einen Griffknopf (86) aufweist, der starr am proximalen Ende des ersten Elementes (12) befestigt und rotierbar an einem stationären Teil der Halterung angebracht ist.

49. Instrument nach Anspruch 48, bei welchem der Griffknopf (86) an dem stationären Teil derart angebracht ist, daß der Griffknopf (86) wahlweise in mehrere diskrete Positionen rotiert werden kann, um dadurch ein wahlweises Positionieren der Öffnung in einer entsprechenden Anzahl diskreter Rotationsorientierungen zu ermöglichen.

## Revendications

1. Instrument chirurgical (10) disposé sensiblement le long d'un axe, ledit instrument chirurgical comprenant :
un premier élément (12) qui s'étend dans une direction distale à partir d'une base (25) et qui comporte une ouverture (13) dans sa région distale, pour admission d'un tissu ;
un deuxième élément (14) disposé à l'intérieur du dit premier élément (12) et comportant une région distale qui tourne en réponse à une force appliquée à une région proximale (30) du dit deuxième élément (14) ;
caractérisé en ce que ledit instrument (10) comprend des moyens de manoeuvre (86) disposés dans une région proximale (62) dudit premier élément, pour changer sélectivement une orientation angulaire dudit premier élément par rapport à ladite base (25).

2. Instrument suivant la revendication 1, comprenant en outre des moyens pour créer dans ledit premier élément (12) une région courbée (18) qui décale angulairement ladite région distale par rapport audit axe, dans une direction choisie ,
ledit premier élément (12) étant relativement flexible, au moins dans ladite région courbée (18), pour permettre auxdits moyens de manoeuvre (86) de changer l'orientation angulaire de ladite ouverture (13) sans changer ladite direction choisie dudit décalage et
au moins une partie dudit deuxième élément (14) qui est située dans ladite région courbée (18) étant relativement flexible pour transmettre ladite force appliquée audit dispositif de coupe (15), par l'intermédiaire de ladite région courbée.

3. Instrument suivant la revendication 2, dans lequel ledit premier élément (12) est évidé dans ladite région courbée (18) pour engendrer la flexibilité relative.

4. Instrument suivant la revendication 2, dans lequel ladite région proximale et ladite région distale dudit premier élément (12) sont rigides, ledit premier élément (12) étant évidé pour engendrer la flexibilité relative.

5. Instrument suivant la revendication 3 ou 4, dans lequel ledit premier élément (12) est évidé par une pluralité d'ouvertures distinctes ménagées dans ses parois.

6. Instrument suivant la revendication 5, dans lequel lesdites ouvertures comprennent une série de fentes axialement espacées et s'étendant circonférentiellement (66).

7. Instrument suivant la revendication 6, dans lequel lesdites fentes pénètrent radialement dans le dit premier élément (12) sur 75% environ du diamètre du dit premier élément.

8. Instrument suivant la revendication 6, dans lequel les fentes adjacentes parmi lesdites fentes (66) pénètrent dans ledit premier élément dans des directions opposées.

9. Instrument suivant la revendication 2, dans lequel ladite extrémité proximale et ladite extrémité distale dudit deuxième élément (14) sont rigides, ladite partie dudit deuxième élément (14) qui est disposée dans ladite région courbée étant évidée pour engendrer la flexibilité relative.

10. Instrument suivant la revendication 9, dans lequel ladite partie dudit deuxième élément (14) est évidée par une série de fentes axialement espacées s'étendant circonférentiellement (34).

11. Instrument suivant la revendication 10, comprenant en outre une matière souple disposée dans au moins certaines desdites fentes (34).

12. Instrument suivant la revendication 9, dans lequel ladite force est fournie par un moteur qui applique un couple à ladite extrémité proximale dudit deuxième élément (14), ladite partie évidée dudit deuxième élément (14) étant configurée pour transmettre ledit couple par l'intermédiaire de ladite région courbée audit dispositif de coupe.

13. Instrument suivant la revendication 2, dans lequel lesdits moyens de création de ladite région courbée comprennent un élément rigide (16) qui est disposé coaxialement audit premier élément (12) et audit deuxième élément (14) et qui est incurvé dans ladite région courbée.

14. Instrument suivant la revendication 13, dans lequel ledit premier élément (12) et ledit deuxième élément (14) sont évidés chacun par une pluralité d'ouvertures dans ladite région courbée,pour engendrer ladite flexibilité, et ledit deuxième élément (14) se déplace par rapport audit premier élément (12) pour déplacer ledit dispositif de coupe (15), ledit élément rigide (16) séparant radialement ledit premier élément et ledit deuxième élément au moins dans ladite région courbée, afin d'éviter une interférence entre ces éléments lorsque ledit deuxième élément se déplace.

15. Instrument suivant la revendication 13, dans lequel ledit deuxième élément (14) est disposé dans ledit élément rigide et ledit élément rigide (16) est disposé dans ledit premier élément (12).

16. Instrument suivant la revendication 15, dans lequel ledit élément rigide (16) comporte une extrémité distale ouverte située du côté proximal dudit dispositif de coupe (15) et de l'ouverture, ledit premier élément (12) et ledit deuxième élément (14) étant configurés pour venir en contact mutuel du côté distal de ladite extrémité, afin de maintenir ledit dispositif de coupe (15) en relation de coupe du tissu avec les bords de la dite ouverture.

17. Instrument suivant la revendication 16, dans lequel une partie de ladite région distale dudit premier élément (12) a un diamètre intérieur réduit par rapport aux autres parties dudit premier élément, afin d'assurer un contact avec ledit deuxième élément.

18. Instrument suivant la revendication 17, dans lequel ladite partie de ladite région distale dudit premier élément (12) bute contre ladite extrémité dudit élément rigide, et ledit diamètre intérieur est le même qu'un diamètre intérieur dudit élément rigide.

19. Instrument suivant la revendication 15, dans lequel au moins ledit premier élément (12) est évidé par une pluralité d'ouvertures (66) dans ladite région coudée, pour engendrer ladite flexibilité, et comprenant en outre une gaine souple disposée sur ledit premier élément dans ladite région courbée, afin de couvrir lesdites ouvertures.

20. Instrument suivant la revendication 1, dans laquelle lesdits moyens de manoeuvre (86) permettent de changer ladite orientation angulaire de ladite ouverture dans une plage d'au moins 180°.

21. Instrument suivant la revendication 20, dans lequel lesdits moyens de manoeuvre (86) permettent de changer ladite orientation angulaire de ladite ouverture dans une plage de 360°.

22. Instrument suivant la revendication 1, dans lequel lesdits moyens de manoeuvre sont configurés pour être actionnés manuellement par un utilisateur du dit instrument afin de changer ladite orientation angulaire de ladite ouverture.

23. Instrument suivant la revendication 22, dans lequel lesdits moyens de manoeuvre comprennent un bouton (86) qui est rigidement fixé à une extrémité proximale dudit premier élément (12) et monté de façon tournante dans une partie fixe de ladite base.

24. Instrument suivant la revendication 23, dans lequel ledit bouton est monté sur ladite partie fixe de sorte que ledit bouton (86) peut être sélectivement tourné à une pluralité de positions distinctes, afin de permettre le positionnement sélectif de ladite ouverture (13) à une pluralité correspondante d'orientations angulaires distinctes.

25. Instrument suivant la revendication 24, dans lequel ladite partie fixe comprend une pluralité de logements dont chacun correspond à une desdites positions distinctes, ledit bouton (86) comprenant un plongeur (106) qui vient sélectivement en prise dans lesdits logements (104) afin de maintenir ladite ouverture dans l'orientation angulaire distincte correspondante.

26. Instrument suivant la revendication 25, dans lequel ledit bouton est élastiquement poussé vers la dite partie fixe pour retenir ledit plongeur (106) dans undit logement (104), afin d'éviter une rotation accidentelle dudit premier élément par rapport à ladite base.

27. Instrument suivant la revendication 23, dans lequel ladite extrémité proximale dudit deuxième élément (14) est fixée à un arbre d'entraînement monté pour un mouvement par rapport à ladite partie fixe et audit bouton.

28. Instrument suivant la revendication 27, dans lequel ledit arbre d'entraînement est agencé pour être entraîné par un moteur afin de faire tourner ledit deuxième élément (14) par rapport audit premier élément (12) et de déplacer ledit dispositif de coupe (15).

29. Instrument suivant la revendication 1, dans lequel ledit deuxième élément (14) est creux et peut recevoir une aspiration à son extrémité proximale et transporter la matière corporelle coupée par ledit dispositif de coupe afin de l'évacuer d'un site chirurgical tandis que l'instrument reste en place pour la poursuite de la coupe.

30. Instrument suivant la revendication 1, dans lequel ledit dispositif de coupe (15) comprend une lame.

31. Instrument suivant la revendication 1, dans lequel ledit premier élément (12) comporte une région courbée qui décale angulairement ladite région distale par rapport audit axe, dans une direction choisie ;
au moins une partie dudit deuxième élément (14), qui est située dans ladite région courbée, étant relativement flexible ; et
ledit premier élément (12) étant relativement flexible, au moins dans ladite région courbée, pour changer sélectivement une orientation angulaire de ladite ouverture par rapport audit axe sans changer ladite direction choisie dudit décalage.

32. Instrument suivant la revendication 31, dans lequel ledit premier élément est évidé,dans ladite région courbée, par une pluralité d'ouvertures distinctes (66) ménagées dans ses parois, pour engendrer la flexibilité relative.

33. Instrument suivant la revendication 31, dans lequel ladite partie dudit deuxième élément est évidée par une pluralité d'ouvertures distinctes (34) ménagées dans ses parois, pour engendrer la flexibilité relative.

34. Instrument suivant la revendication 32, dans lequel ladite force est fournie par un moteur qui applique un couple à l'extrémité proximale dudit deuxième élément (14), ladite partie dudit deuxième élément (14) étant configurée pour transmettre ledit couple par l'intermédiaire de ladite région courbée audit dispositif de coupe (15).

35. Instrument suivant la revendication 31, dans lequel lesdits moyens de création de ladite région courbée comprennent un élément rigide (16) qui est disposé coaxialement audit premier élément (12) et audit deuxième élément (14) et qui est incurvé dans ladite région courbée.

36. Instrument suivant la revendication 35, dans lequel ledit premier élément (12) et ledit deuxième élément (14) sont évidés chacun par une pluralité d'ouvertures (66, 34) dans ladite région courbée, pour engendrer ladite flexibilité, et ledit deuxième élément tourne par rapport au dit premier élément pour déplacer ledit dispositif de coupe, ledit élément rigide (16) séparant radialement ledit premier élément et ledit deuxième élément au moins dans ladite région coudée, afin d'éviter une interférence entre ces éléments lorsque ledit deuxième élément tourne.

37. Instrument suivant la revendication 35, dans lequel ledit deuxième élément (14) est disposé dans ledit élément rigide (16) et ledit élément rigide est disposé dans ledit premier élément (12).

38. Instrument suivant la revendication 31, dans lequel lesdits moyens de manoeuvre sont configurés pour être actionnés manuellement par un utilisateur dudit instrument, afin de changer ladite orientation angulaire de la dite ouverture.

39. Instrument suivant la revendication 38, dans lequel lesdits moyens de manoeuvre comprennent un bouton (86) qui est rigidement fixé à une extrémité proximale dudit premier élément (12) et monté de façon tournante sur une partie fixe de ladite base.

40. Instrument suivant la revendication 39, dans lequel ledit bouton (86) est monté sur ladite partie fixe de sorte qu'on peut faire tourner sélectivement ledit bouton à une pluralité de positions distinctes afin de permettre le positionnement sélectif de ladite ouverture à une pluralité correspondante d'orientations angulaires distinctes.

41. Instrument chirurgical suivant la revendication 1 ou 2, dans lequel ledit instrument comprend en outre :
un tube support fixe (16) qui s'étend dans la direction distale à partir d'une base et qui est disposé coaxialement entre lesdits premier et deuxième éléments (12, 14).

42. Instrument suivant la revendication 41, dans lequel lesdits premier et deuxième éléments (12, 14) et ledit tube support (16) sont évidés chacun dans ladite région courbée, afin d'engendrer la flexibilité relative.

43. Instrument suivant la revendication 42, dans lequel ledit premier élément (12) et ledit deuxième élément (14) sont évidés chacun par une pluralité d'ouvertures distinctes (66,34) ménagées dans leurs parois.

44. Instrument suivant la revendication 41, dans lequel ladite force est fournie par un moteur qui applique un couple à ladite extrémité proximale dudit deuxième élément (14), ladite partie dudit deuxième élément (14) étant évidée pour engendrer la flexibilité relative et étant configurée pour transmettre ledit couple par l'intermédiaire de ladite région courbée audit dispositif de coupe.

45. Instrument suivant la revendication 41, dans lequel ledit tube support (16) présente une extrémité distale ouverte située du côté proximal dudit dispositif de coupe et de ladite ouverture, ledit premier élément et ledit deuxième élément étant configurés pour venir en contact mutuel du côté distal de ladite extrémité, afin de maintenir ledit dispositif de coupe (15) en relation de coupe du tissu avec les bords de ladite ouverture.

46. Instrument suivant la revendication 45, dans lequel une partie de ladite région distale dudit premier élément (12) a un diamètre intérieur réduit par rapport aux autres parties dudit premier élément, afin d'assurer ledit contact avec ledit deuxième élément (14).

47. Instrument suivant la revendication 41, dans lequel lesdits moyens de manoeuvre (86) sont configurés pour être actionnés manuellement par un utilisateur dudit instrument, pour changer ladite orientation angulaire de ladite ouverture.

48. Instrument suivant la revendication 47, dans lequel lesdits moyens de manoeuvre comprennent un bouton (86) qui est rigidement fixé à une extrémité proximale dudit premier élément (12) et qui est monté de façon tournante dans une partie fixe de ladite base.

49. Instrument suivant la revendication 48, dans lequel ledit bouton (86) est monté dans ladite partie fixe de sorte qu'on peut faire tourner sélectivement ledit bouton (86) à une pluralité de positions distinctes, afin de permettre le positionnement sélectif de ladite ouverture à une pluralité correspondante d'orientations angulaires distinctes.
